# EUROPEAN PATENT APPLICATION

(11) **EP 3 936 059 A2**
(43) Date of publication of application: **12.01.2022**
(21) Application number: 21181367.0
(22) Date of filing: 24.06.2021
(51) Int. Cl.: A61B 17/04

(54) **PERCUTANEOUS DEVICE FOR CLOSING A BLOOD VESSEL**

(30) Priority: 07.07.2020 IT 202000016426
(71) Applicant: Bergamaschi, Gastone, 37121 Verona (IT)
(72) Inventor: Bergamaschi, Gastone, 37121 Verona (IT)
(74) Representative: Gualeni, Nadia

(57) **Abstract**

The percutaneous device (100) for closing a blood vessel according to the invention comprises: a main body (1) in which a distal clip (2) and a proximal clip (3) which are connected to each other by means of a tie rod (4) are accommodated and an abutment element (5) for the clips (2, 3) is accommodated; and a handle (7) for maneuvering the clips (2, 3), the abutment element (5) and the tie rod (4). This device is characterized in that the distal clip (2) is provided at least with a proximal housing (22), and the proximal clip (3) is provided with both a distal housing (32) and a proximal housing (33), and wherein the tie rod (4) is positioned inside said housings (22, 32, 33) when the clips (2, 3) are inserted inside the main body (1), so as to not interfere with the correct sliding thereof.

## Description

The subject of the present invention is a device for interrupting the function of superficial venous collectors, using percutaneous access and ultrasonic guidance.

Surgical treatment for superficial venous insufficiency of the lower limbs has seen dramatic development in recent decades. The widespread need for less invasive surgery, together with technological evolution, has resulted in the most traumatic procedures (such as the Linton operation) becoming completely obsolete and standard procedures (such as saphenous vein stripping) being greatly scaled back, and has resulted in low-invasive procedures being used, such as the ablation of superficial venous collectors by means of thermal techniques (laser; radiofrequency) or non-thermal techniques (sclerosis foam; mechanochemical ablation; cyanoacrylate glues), in addition to surgical strategies such as endoscopic subfascial ligation of perforating veins or saphenous reflux segmentation.

Some of the aforementioned known techniques are intended for well-defined niche pathologies, whereas others are, in the absence of a clear hierarchy, valid alternative therapies in terms of efficacy, reduction of complications, long-term results and costs.

It is nevertheless noted that the "surgical" techniques are linked to invasiveness and a significant risk of complications, while the endoluminal ablative techniques entail a share of long-term failures (recanalizations) and involve a likelihood, albeit very small, of serious complications. With regard to the aforementioned ablative techniques, it is widely believed that the point of maximum criticality in the methodology can be found in the sapheno-femoral confluence, i.e. the short venous site which, in the event of over- or under-treatment, may place the patient at risk of thrombosis that extends to deep circulation or central embolism and at risk of more or less extensive recanalization of the vessel by blood reflux, with recurrence of the varicose syndrome.

In the field of treating superficial venous insufficiency of the lower limbs, there is therefore a strong need to carry out a procedure which is to all effects "surgical," such as interruption in order to ligate a venous collector, but without necessarily exposing the patient to the invasiveness and the preventable complications involved therewith.

The object of this invention is to provide a percutaneous device for interrupting the function of venous collectors that makes it possible to mechanically occlude a venous vessel by means of transcutaneous puncture access.

This object is achieved by a percutaneous device for interrupting the function of venous collectors according to claim 1. Other embodiments of the percutaneous device according to the invention are described in the dependent claims.

The device according to this invention makes it possible to carry out operations to intercept and/or close a blood vessel using percutaneous access in a simplified manner, which requires reduced manual ability which is not necessarily surgical. As a result, the operating times and the impact on patient approval acceptance (or compliance) are drastically reduced by comparison with "open" surgery techniques.

Further advantages of a percutaneous device according to this invention will become apparent from the following description, given by way of non-limiting example and in accordance with the accompanying figures, in which:
- Fig. 1A shows a device according to this invention in a configuration for percutaneous insertion;
- Fig. 1B shows a device according to this invention in a configuration for partial release of the distal clip;
- Fig. 1C shows a device according to this invention in a configuration for release of the distal clip;
- Fig. 1D shows a device according to this invention in a configuration for partial release of the proximal clip;
- Fig. 1E shows a device according to this invention in a configuration for release of the proximal clip;
- Fig. 1F shows a device according to this invention in configuration for closing the venous vessel;
- Fig. 2A and 2B show a device according to this invention in one embodiment comprising a cylindrical tie rod:

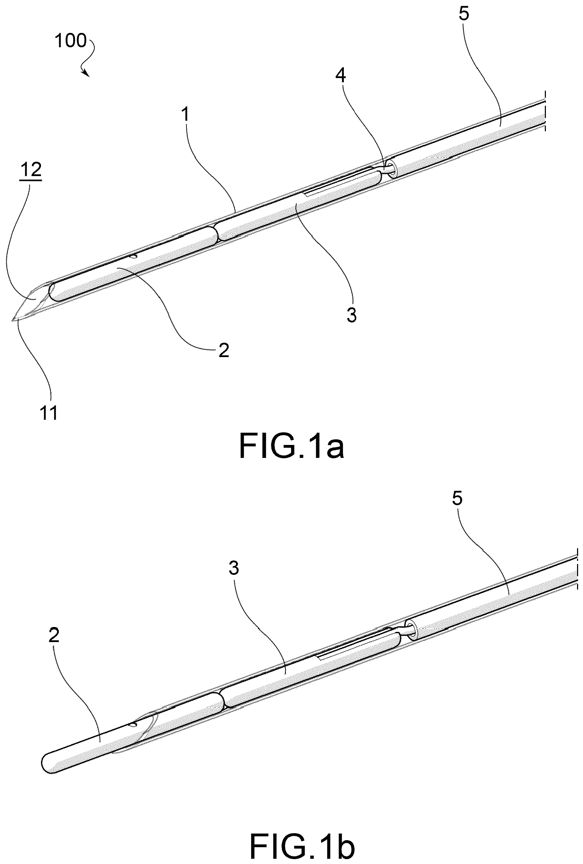

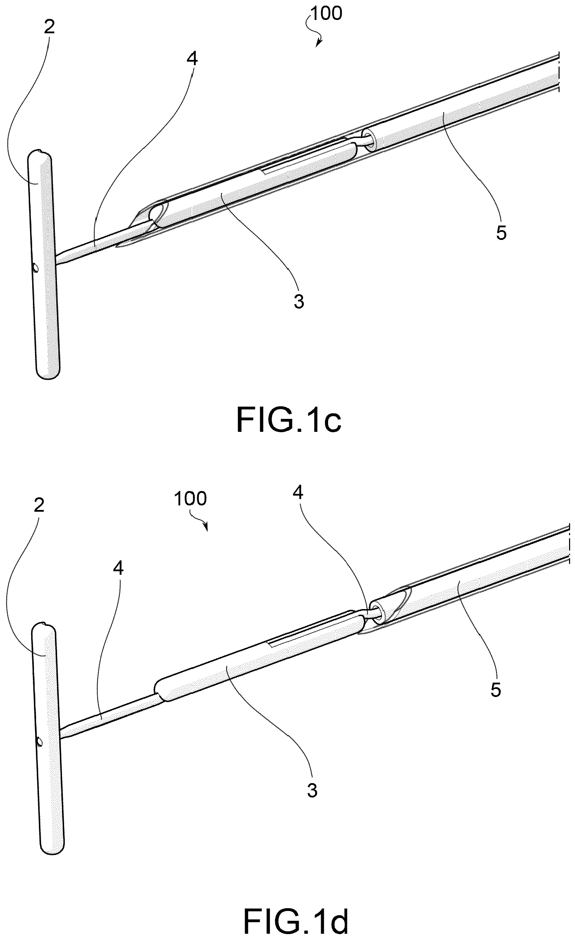

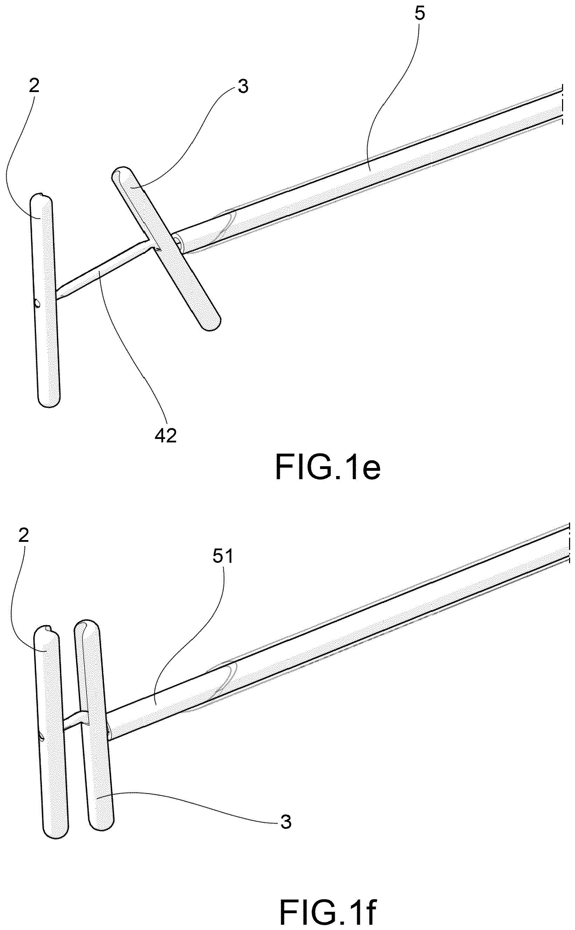

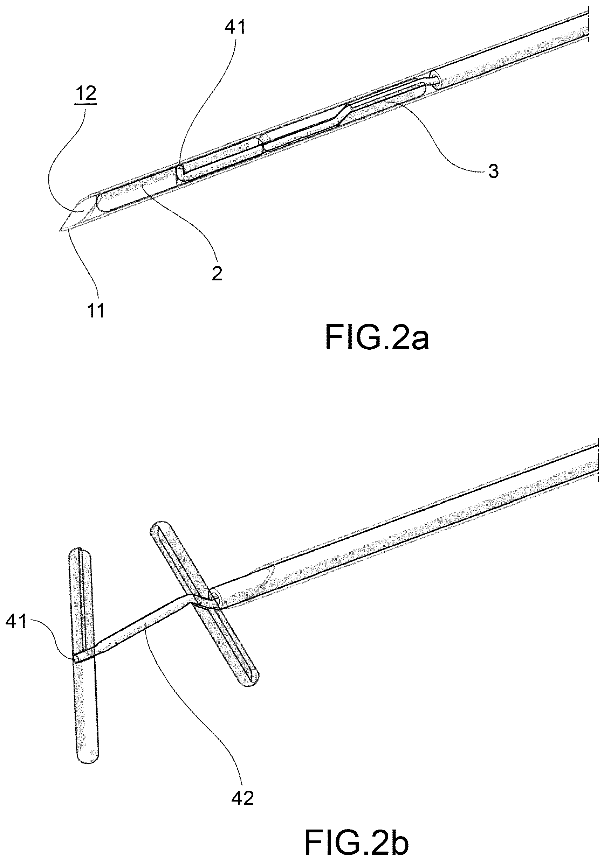

- Fig. 2A is a partial transparent view of the device as in Fig. 1A in order to better visualize the arrangement of the tie rod of the clips;
- Fig. 2B is a partial transparent view of the device as in Fig. 1E in order to better visualize the arrangement of the tie rod after the clips have been released;

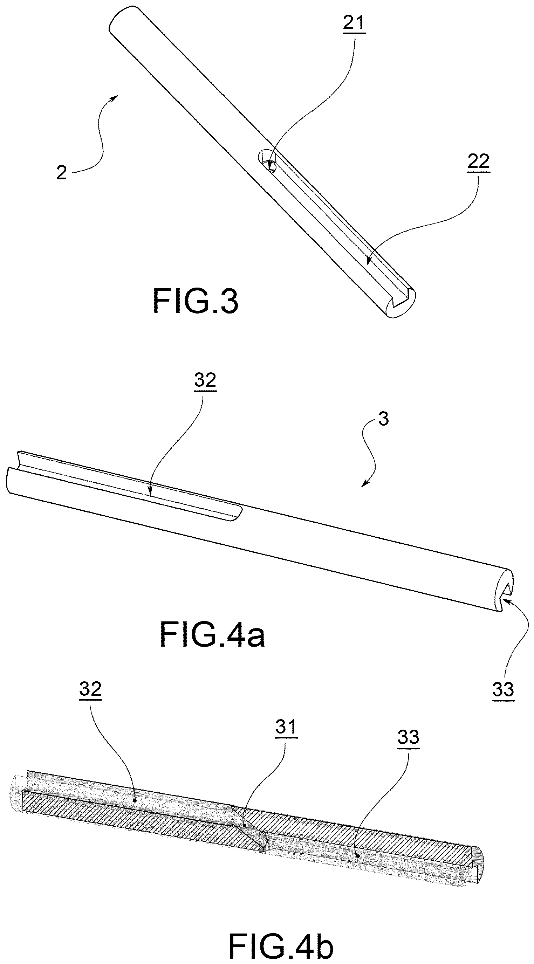

- Fig. 3 is an axonometric view of the distal clip in one embodiment comprising a slot which is adapted to receive the tie rod element and has a U-shaped cross section;
- Fig. 4A and 4B are an axonometric view and a cross-sectional view, respectively, of the proximal clip in one embodiment;

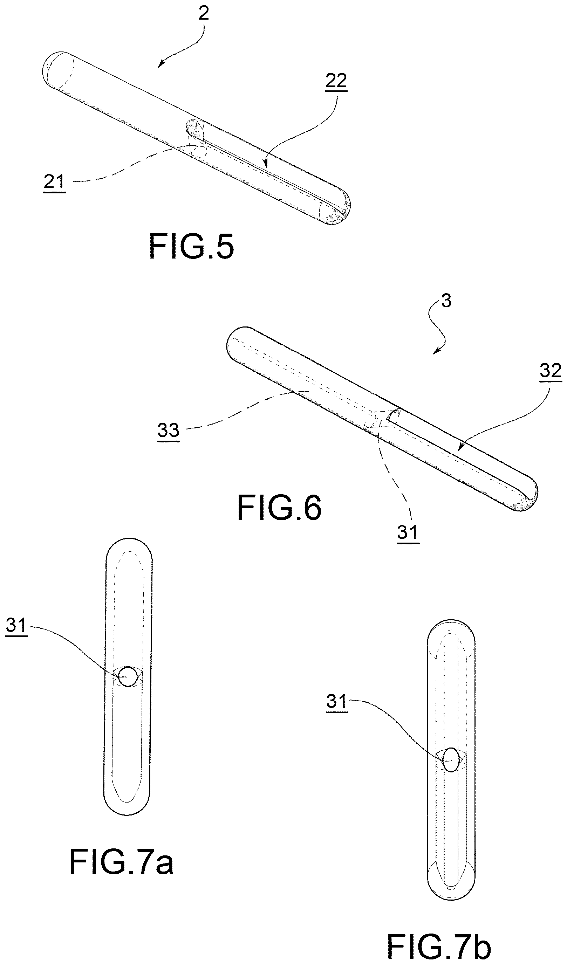

- Fig. 5 is an axonometric view of the distal clip in a further embodiment comprising a slot which is adapted to receive the tie rod element and has a V-shaped cross section;
- Fig. 6 is an axonometric view of the proximal clip in a further embodiment;
- Fig. 7A and 7B show two embodiments of the hole in the proximal clip;

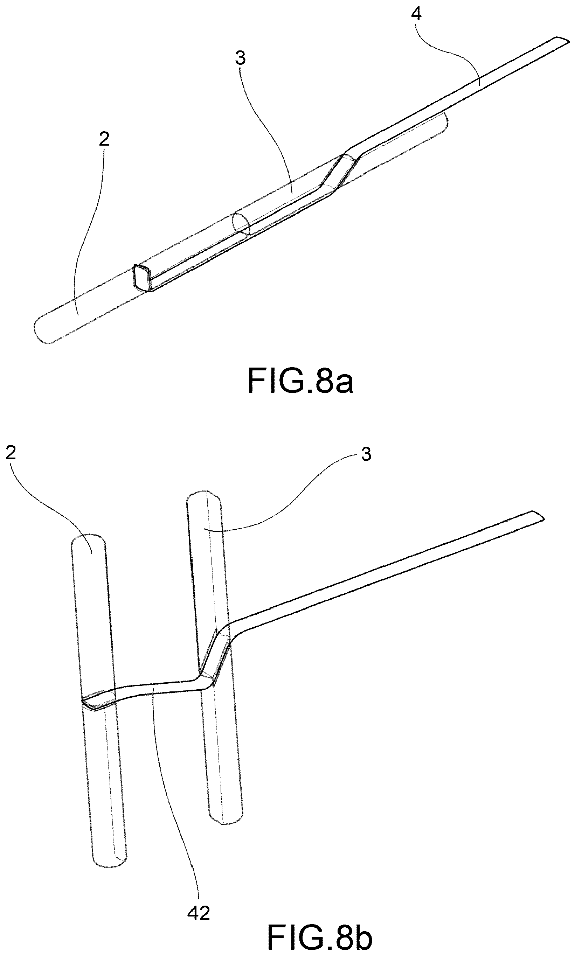

- Fig. 8A and 8B show a device according to this invention in one embodiment comprising a flattened tie rod:
- Fig. 8A is a partial transparent view of the device as in Fig. 1A in order to better visualize the arrangement of the tie rod of the clips;
- Fig. 8B is a partial transparent view of the device as in Fig. 1E in order to better visualize the arrangement of the tie rod of the clips;

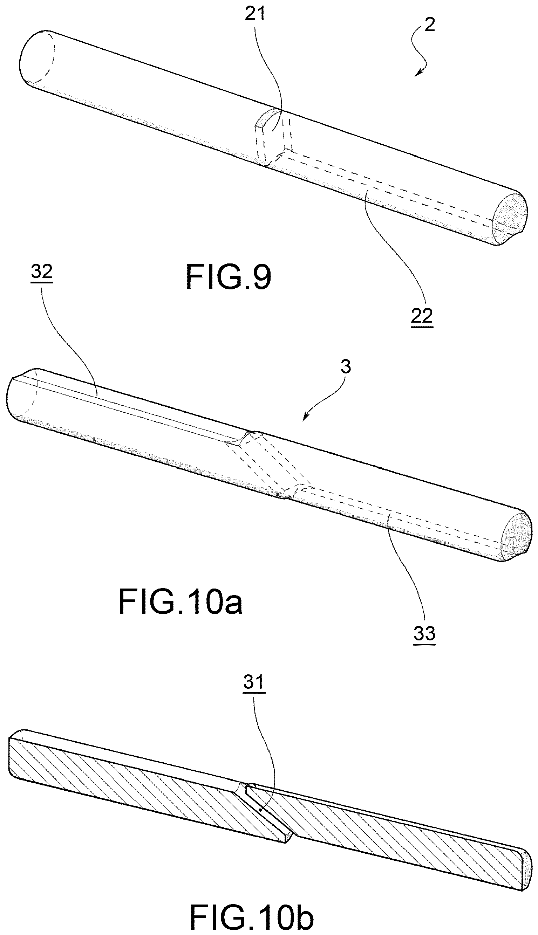

- Fig. 9 is an axonometric view of the distal clip in a further embodiment;
- Fig. 10A and 10B are an axonometric view and a cross-sectional view, respectively, of the proximal clip in a further embodiment;

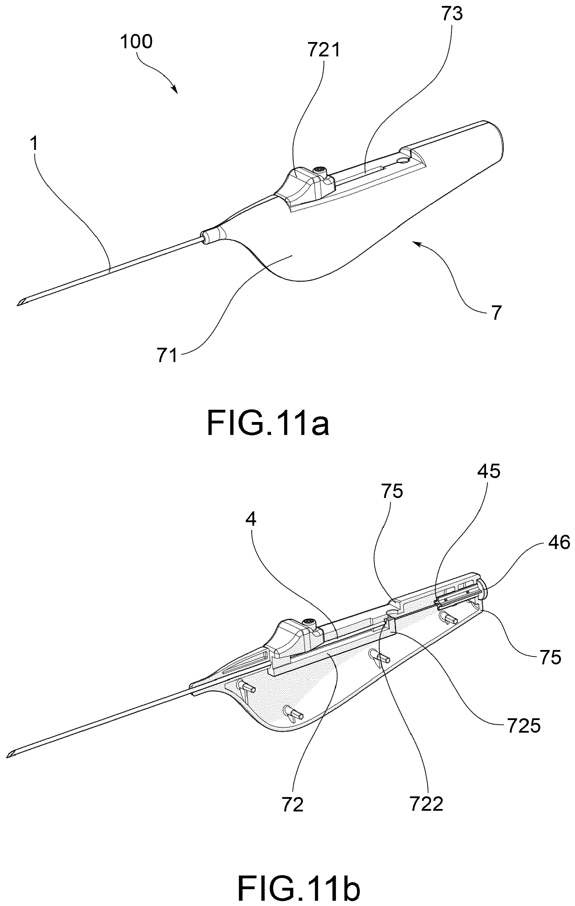

- Fig. 11A and 11B are an axonometric view and a cross-sectional view, respectively, of a device according to this invention that is provided with a handle in one embodiment;

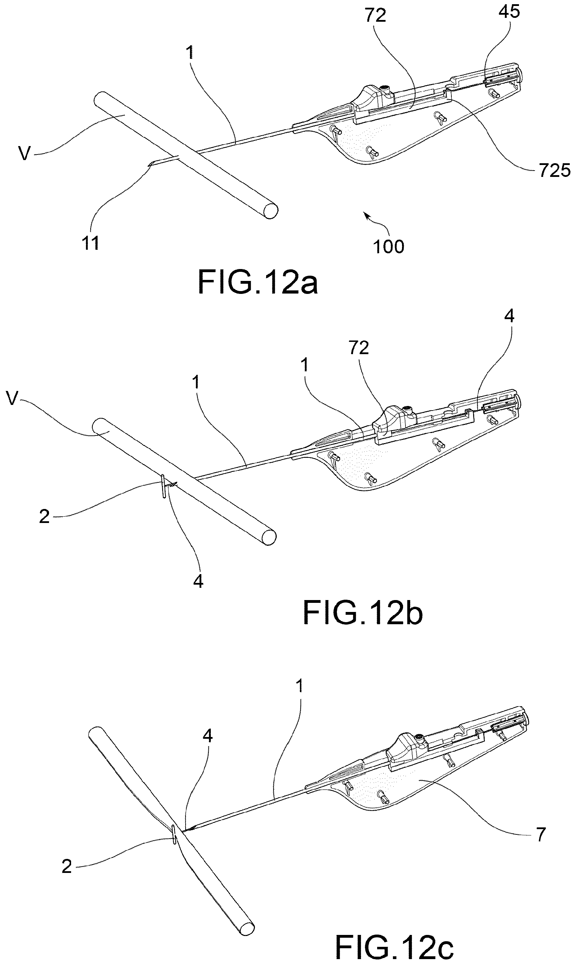

- Fig. 12A to 12F show the steps of the process for closing a venous vessel by means of the device in Fig. 11A, in particular:
- 12A: puncturing the venous vessel;
- 12B: releasing the distal clip;
- 12C: pulling the distal clip against the vessel;
- 12D: releasing the proximal clip;
- 12E: pushing the proximal clip against the vessel;
- 12F: clamping the clips and closing the vessel;

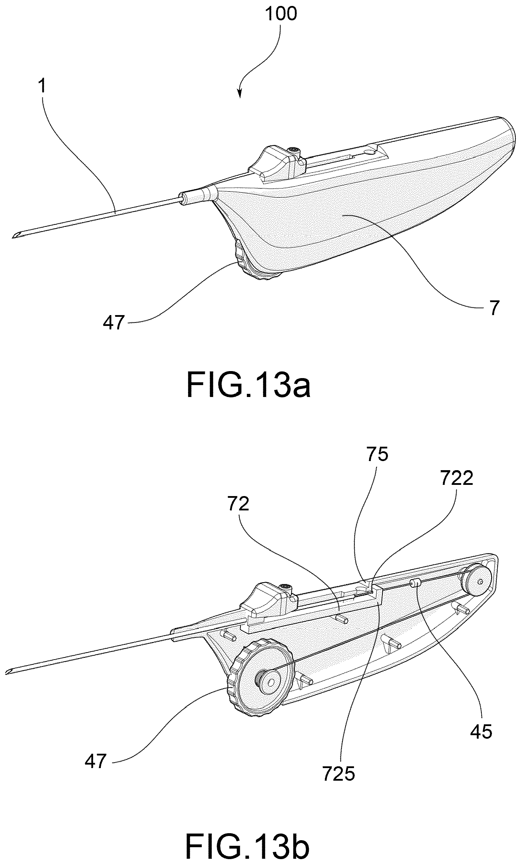

- Fig. 13A and 13B are an axonometric view and a cross-sectional view, respectively, of a device according to this invention provided with a handle in a further embodiment;

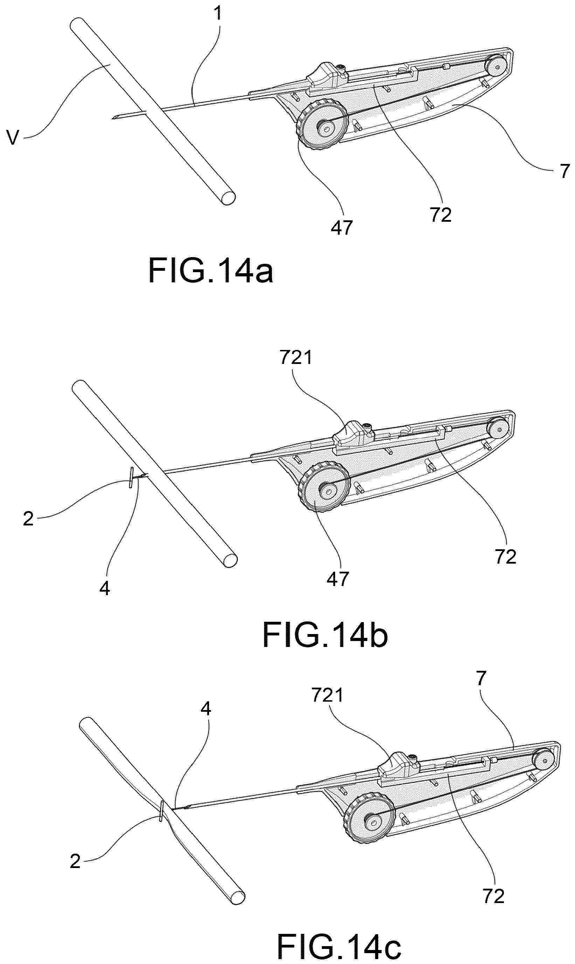

- Fig. 14A to 14F show the steps of the process for closing a venous vessel by means of the device in Fig. 13A, in particular:
- 14A: puncturing the venous vessel;
- 14B: releasing the distal clip;
- 14C: pulling the distal clip against the vessel;
- 14D: releasing the proximal clip;
- 14E: pushing the proximal clip against the vessel;
- 14F: clamping the clips and closing the vessel;

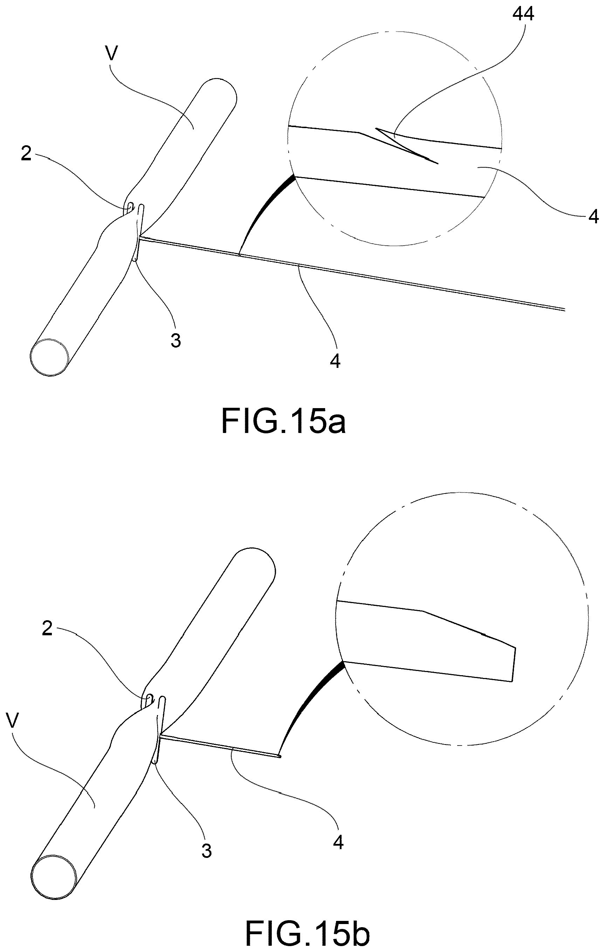

- Fig. 15A and 15B show the final steps of the process for closing a venous vessel, in particular:
- 15A: weakening the tie rod of the clips;
- 15B: breaking the tie rod in order to completely release the clips;

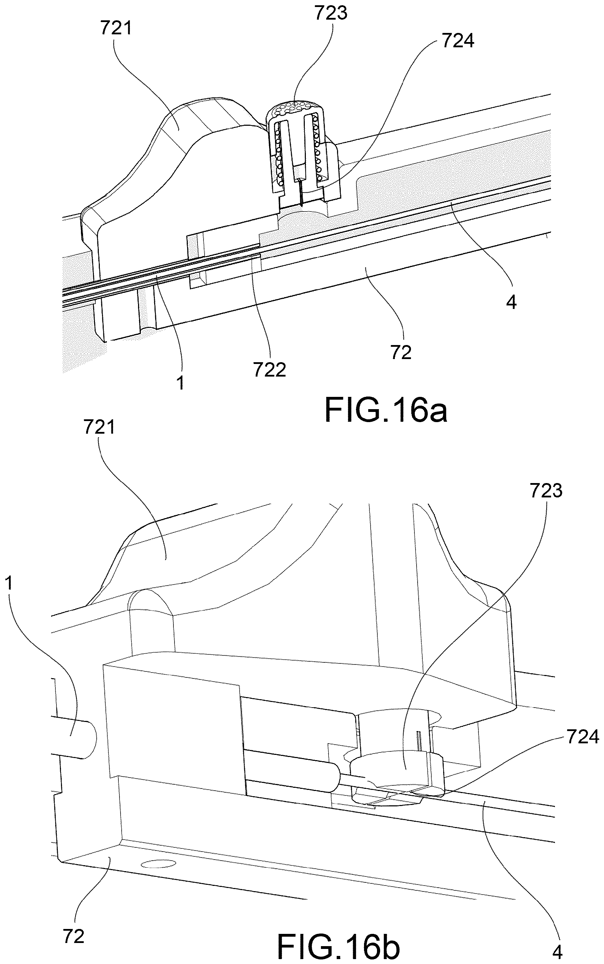

- Fig. 16A and 16B are detailed views of the proximal cutting system for cutting the tie rod of the clips:
- 16A: deactivated cutting system;
- 16B: activated cutting system.

In the aforementioned figures, similar components are identified in different figures by the same reference sign.

Reference sign 100 represents a percutaneous device for interrupting the function of superficial venous collectors according to this invention. This device is suitable for intercepting and closing a blood vessel, for example the saphenous vein.

The device 100 comprises a main body 1 which extends along a longitudinal axis X and is internally hollow in order to house a pair of clamping clips 2, 3 and an abutment element 5 for the clips 2, 3.

The main body 1 is preferably a metal cannula which may be inserted percutaneously, and is provided with a distal perforation tip 11, for example in the form of a needle. The distal tip 11 comprises a distal opening 12 from which the clips 2, 3 exit.

The abutment element 5 is preferably a rod which is cylindrical, elliptical or semi-circular.

The abutment element 5 extends along the longitudinal axis X and is provided with a passageway for the tie rod 4. The passageway for the tie rod 4 is, for example, a central hole such that the tie rod 4 slides internally in the abutment element 5, or a lateral groove such that the tie rod 4 slides externally to the abutment element 5. The abutment element 5 is provided with a distal end 51 adapted to push the proximal clip 3.

The device 100 preferably comprises a handle 7 which is connected to the proximal portion of the main body 1 and is adapted to maneuver the clips 2, 3 and the tie rod 4.

The overall configuration of the device 100 is such that the pair of clamping clips 2, 3 and the abutment element 5 may be slidably inserted inside the main body 1, may exit from the distal opening 12, and may be maneuvered from the outside by means of the handle 7. It should be noted that the abutment element 5 is integral with the handle 7.

The pair of clips comprises a distal clip 2 for intercepting the vessel and a proximal clip 3 for clamping the vessel.

The clips 2, 3 are preferably substantially in the form of a bar or rod, i.e. have a cylindrical and elongate shape.

Each clip 2, 3 preferably has a length determined by the specific surgical requirements and by the dimensions of the vessel to be treated, for example in a range of approximately 6-12 mm.

The clips 2, 3 may be the same length or may be different lengths.

The clips 2, 3 are preferably made of biocompatible metal material or made of bioresorbable material.

The clips 2, 3 are connected to each other by means of a tie rod 4 or an equivalent means.

In one embodiment, shown in Fig. 2A and 2B, the tie rod 4 has a substantially circular or semi-circular cross section.

In a further embodiment, shown in Fig. 8A and 8B, the tie rod 4 has a flattened cross section or is substantially a tape.

In yet a further embodiment, the tie rod 4 has a flattened cross section distally, preferably between the two clips 2, 3, and then continues with a circular cross section proximally. In this embodiment, the tie rod 4 has a flattened cross section for a distal length between the length of half a clip and the length of two clips.

The tie rod 4 is preferably made of nylon or polyester or is made of resorbable material.

The clips 2, 3 are provided with a seat 21, 31 into which the tie rod 4 is inserted.

The distal clip 2 is provided with a distal seat 21 into which the tie rod 4 is fastened.

The distal seat 21 is preferably a through hole which has a circular (Fig. 3 and 5) or rectangular cross section.

The distal seat 21 preferably transversely crosses the distal clip 2 perpendicularly. This solution makes it possible to correctly orient and turn over the distal clip 2 once it has been released.

The proximal clip 3 is provided with a proximal seat 31 into which the tie rod 4 is slidably inserted.

The proximal seat 31 is preferably a through hole which has a circular (Fig. 7A) or oval (Fig. 7B) or rectangular (Fig. 10A) cross section.

The proximal seat 31 preferably transversely crosses the proximal clip 3 obliquely. This solution makes it possible both to correctly orient the proximal clip 3 once it has been released and to correctly slide the tie rod 4.

In particular, a distal end 41 of the tie rod 4 is fastened in the seat 21 of the distal clip 2, the tie rod 4 is also slidably inserted in the seat 31 of the proximal clip 3 such that an intermediate portion 42 of the tie rod 4 is arranged between the clips 2, 3, and a proximal end 43 of the tie rod 4 may be maneuvered by the surgeon, either directly or by means of a handle 7.

Depending on the specific application, the tie rod 4 and the clips 2, 3 may be made of non-resorbable or resorbable material.

The clips 2, 3 preferably have transverse dimensions (i.e. a diameter) so as to be able to slide freely, possibly with minimal friction in order to prevent slipping, inside the main body 1.

When the clips 2, 3 are inserted inside the main body 1, the longitudinal axis of the clips 2, 3 is in line with the longitudinal axis of the main body 1.

One essential aspect for the correct functioning of the device 100 is the positioning of the tie rod 4 inside the main body 1, which tie rod must not interfere with the correct sliding of the clips 2, 3.

The clips 2, 3 are therefore provided with at least one housing 22, 32, 33 in which the tie rod 4 is positioned when the clips 2, 3 are inside the main body 1.

The housing 22, 32, 33 is a longitudinal groove formed on the outer surface of the clip 2, 3 such that the tie rod 4 is inserted into this groove and therefore fits inside the volume of space occupied by the clip 2, 3, as may be seen in Fig. 1A, 2A and 8A.

The distal clip 2 is provided with a proximal housing 22, and optionally also a distal housing for fastening the tie rod.

The proximal clip 3 is provided with both a distal housing 32 and a proximal housing 33.

It should be noted that the distal housing and the proximal housing are arranged on opposite sides of the clip.

In one embodiment, shown in Fig. 3 and 4A, the housing 22, 32, 33 has a rectangular or square or U-shaped cross section.

In one embodiment, shown in Fig. 5 and 6, the housing 22, 32, 33 has a triangular or V-shaped cross section.

In one embodiment, shown in Fig. 9 and 10A, the housing 22, 32, 33 is a longitudinal flattened portion or a C-shaped or crescent-shaped recess.

The housing 22, 32, 33 comprises an inlet end to the clip 2, 3 and an end that traverses the clip 2, 3 and flows into the seat 21, 31.

The distal clip 2 therefore comprises a path for the tie rod 4 that starts from the seat 21 and continues along the housing 22. The path for the tie rod 4 occupies approximately half of the length of the distal clip 2.

In one embodiment, the distal clip 2 also comprises a distal housing used as a further compartment (in addition to the seat 21) for fastening the tie rod 4. In this example, therefore, the distal clip 2 comprises a path for the tie rod 4 that starts from the distal housing and continues into the seat 21 through the clip in order to exit from the opposite side and continue along the housing 22. In this case, the path for the tie rod 4 therefore occupies more than half of the length of the distal clip 2.

The proximal clip 3 comprises a path for the tie rod 4 that starts from the housing 32 and continues into the seat 31 through the clip in order to exit from the opposite side and continue along the housing 33. The path for the tie rod 4 occupies the entire length of the proximal clip 3.

In the embodiment shown in Fig. 8A and 8B in which the tie rod 4 has a flattened cross section, the seat 21, 31 into which the tie rod 4 is inserted is rectangular and may be arranged longitudinally or transversely with respect to the clip 2, 3. Moreover, in this embodiment, the housing 22, 32, 33 in which the tie rod 4 is positioned when the clips 2, 3 are inside the main body 1 may be C-shaped or crescent-shaped.

The device 100 comprises a handle 7 which extends predominantly longitudinally, is formed by two half-shells mechanically connected together, and is connected to the proximal portion of the main body 1.

The handle 7 comprises a handle body 71 provided with a track 73 in which an operating lever 72 for operating the clips 2, 3 and the tie rod 4 is slidably accommodated.

The operating lever 72, which may be seen in detail in Fig. 16A, extends predominantly longitudinally and preferably comprises a slider 721 which is shaped so as to facilitate gripping said slider and sliding it inside the track 73.

The main body 1 is integral with the operating lever 72, which means that retracting the operating lever 72 also retracts the main body 1, as for example in Fig. 12B and 14B.

The operating lever 72 comprises at least one seat 722 for the passageway of the tie rod 4, such that the lever may slide on the tie rod 4.

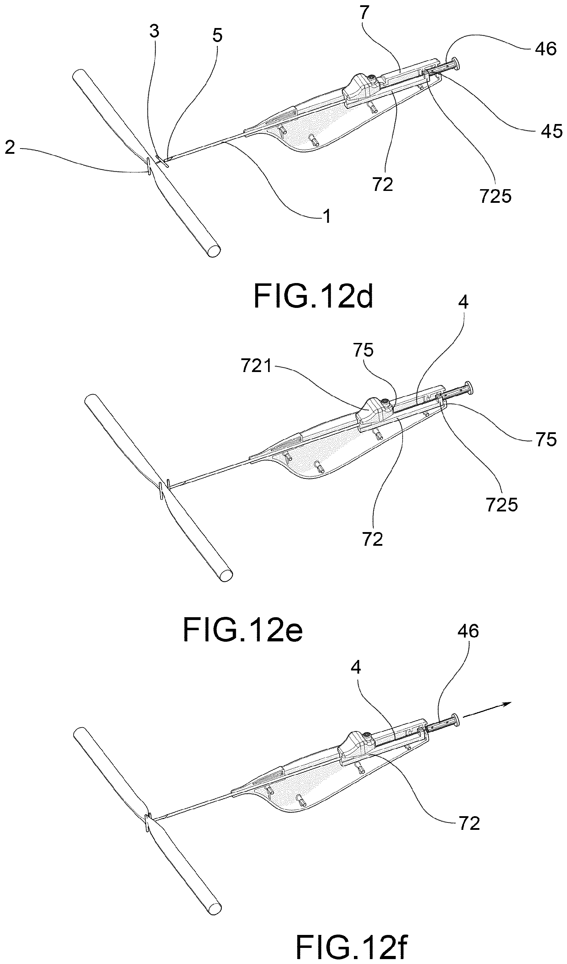

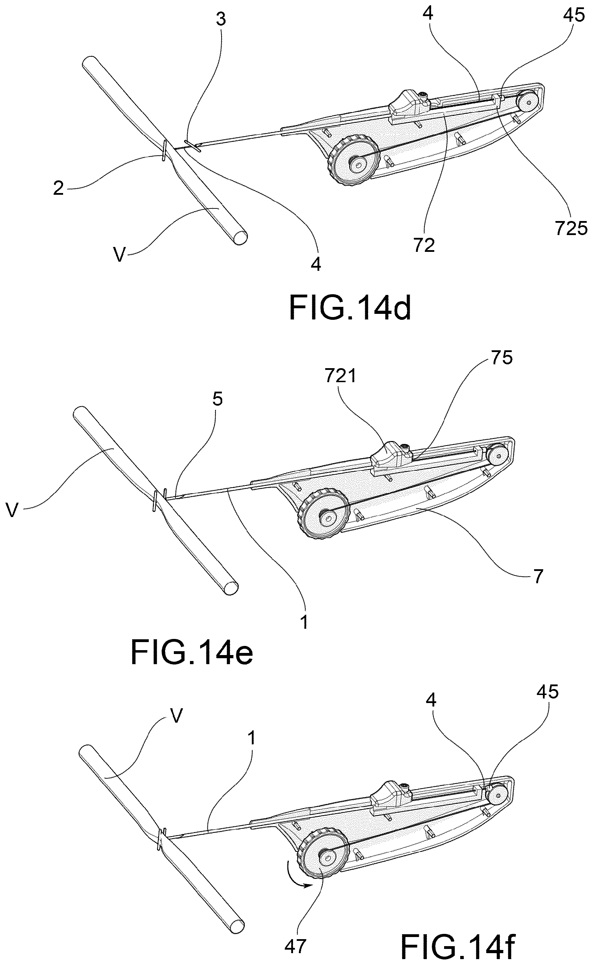

The operating lever 72 comprises, at the rear, a lever stop 725 adapted to abut against a relevant tie rod stop 45 such that the operating lever 72 becomes integral with the tie rod 4 and such that retracting the operating lever 72 also retracts the tie rod 4, as may be seen in Fig. 12D and 14D.

The handle 7 comprises a handle stop 75 adapted to abut against the lever stop 725 or against the slider 721 such that the operating lever 72 may not be retracted any further, as may be seen in Fig. 12E and 14E.

The tie rod 4 is also provided with a traction element adapted to pull the tie rod 4 with respect to the handle 7.

In the variant in Fig. 11B, the traction element 45 is connected to the proximal end 43 of the tie rod 4. The traction element 45 at least partially exits the handle 7 in order to be gripped and optionally pulled by the medical professional, as in Fig. 12F.

In the variant in Fig. 13B, the traction element is a wheel 47 connected to the proximal end 43 of the tie rod 4. The wheel 47 at least partially exits from the handle 7 in order to be optionally rotated by the medical professional, as in Fig. 14F.

The operating lever 72 comprises, in proximity to the slider 721, a cutting button 723 provided with a blade 724 adapted to sever the tie rod 4. The cutting button 723 is vertically slidable between a rest position (Fig. 16A) in which the blade 724 is spaced apart from the tie rod 4 and a cutting position (Fig. 16B) in which the blade 724 is pushed against the tie rod 4 in order to sever it.

In one embodiment shown in Fig. 15A, the tie rod 4 is provided, proximally with respect to the proximal clip 3, with a predetermined breaking point 44, for example a pre-incision or a weakened portion, that is adapted to yield in response to a certain traction force exerted on the tie rod 4. Said predetermined breaking point 44 is a calibrated point of weakness that makes it possible to only release the pair of clips after an optimal traction force has been exerted, in order to clamp and therefore occlude the vessel. This solution allows as little of the tie rod 4 as possible to be left inside the body of the patient after the vessel has been closed, as shown in Fig. 15B.

The sequence of figures from 12A to 12F and from 14A to 14F shows the use of the device 100 in order to close a blood vessel.

As shown in Fig. 12A and 14A, firstly the main body 1 is introduced percutaneously by means of the needle tip 11. The tip 11 then punctures the wall of the blood vessel V and passes through said vessel from one side to the other. During insertion of the device 100, the longitudinal axis of the clips 2, 3 is in line with the longitudinal axis of the main body 1.

At this point, as shown in Fig. 12B and 14B, the distal clip 2, and with it also part of the tie rod 4, is made to exit from the distal opening 12 in the main body 1. The distal clip 2 is made to exit by retracting the operating lever 72 and the main body 1 therewith, while keeping the proximal clip 3 and the abutment element 5 in position.

The tie rod 4 is then pulled, as may be seen in Fig. 12C and 14C, by pulling the handle 7, in order to correctly position the distal clip 2 against the outer wall of the vessel V. At this point, the distal clip 2 is arranged externally and substantially transversely with respect to the main body 1. Ultrasonic checks are used to verify that the distal clip 2 is abutting transversely against the distal wall of the blood vessel V.

At this point, as shown in Fig. 12D and 14D, the proximal clip 3, and with it also part of the tie rod 4, is also made to exit the distal opening 12 in the main body 1 in the same manner as the distal clip 2. The proximal clip 3 is made to exit by retracting the operating lever 72 and the main body 1 therewith, while keeping the abutment element 5 in position.

The tie rod 4 is then pulled, as may be seen in Fig. 12E and 14E, by pulling the handle 7, in order to correctly position the proximal clip 3 against the outer wall of the vessel V. At this point, the proximal clip 3 is also arranged externally and substantially transversely with respect to the main body 1.

At this point, the tie rod 4 is also pulled, as may be seen in Fig. 12F and 14F, in order to correctly clamp the vessel between the distal clip 2 and the proximal clip 3. The tie rod 4 is optionally pulled by means of the traction element 45 or by rotating the wheel 47.

It should be noted that the tie rod slides into the seat 21 of the proximal clip 3 with a calibrated friction that allows the clips 2, 3 to clamp.

The tie rod 4 is preferably provided with a radial dimension, for example a flattened portion or a thickened portion in a proximal position, so as to prevent the tie rod from being able to reenter the seat 31 of the proximal clip 3 once this radial dimension has exited therefrom. This solution makes it possible to maintain the correct clamping of the vessel between the distal clip 2 and the proximal clip 3, thus avoiding the need to produce a node on the tie rod that has to be pushed into position.

At this point, it is possible to sever the tie rod 4 by means of actuating the cutting button 723 present on the handle or by means of pulling further if a predetermined breaking point 44 on the tie rod 4 is used.

A percutaneous device according to this invention innovatively makes it possible to carry out operations to intercept and/or close a blood vessel percutaneously in a simplified manner, which requires reduced manual ability which is not necessarily surgical.

In the percutaneous device according to this invention, as a result of the housings 22, 32, 33 and the respective paths provided on the clips 2, 3, the tie rod 4 advantageously does not interfere with the correct sliding of the clips 2, 3 inside the main body 1.

The percutaneous device according to this invention advantageously makes it possible to implement a mechanical method for closing a vein, or in general a blood vessel, even a large-caliber vessel, by means of closing or "clamping" on the outer wall of the blood vessel, which method is carried out percutaneously. It is therefore possible to perform ultrasound-guided obstruction of a vein by means of percutaneous clamping.

Advantageously, as a result of using a percutaneous device according to this invention, the operating times and the impact on patient approval acceptance (or compliance) are drastically reduced by comparison with "open" surgery techniques.

The clips 2, 3 may advantageously be made of resorbable material, and may remain in position in order to guarantee the interruption of the venous blood flow while not interrupting the anatomical continuity of the vascular structure.

The device according to this invention advantageously has various fields of application such as: superficial venous insufficiency; any case in which it is necessary to "ligate" or interrupt a venous tract; sapheno-femoral crossectomy; sapheno-popliteal crossectomy; interruption of varicose collaterals; interruption of incontinent perforating veins; segmentation of the saphenous reflux.

The process may advantageously be intended as a simple isolated surgical procedure carried out on one or more sites, or may be a process carried out in association with other operations. An example of this is the interruption of the sapheno-femoral junction carried out in association with a procedure to ablate the great saphenous vein that is carried out using physical or chemical techniques.

It is understood that a person skilled in the art could make modifications to the product described above, all of which are contained within the scope of protection as defined by the following claims.

## Claims

1. A percutaneous device (100) for closing a blood vessel, comprising:
- an internally hollow main body (1) provided with a distal perforation tip (11) having a distal opening (12);
- a distal clip (2) and a proximal clip (3) slidably accommodated inside the main body (1), said clips (2, 3) being connected to each other by means of a tie rod (4); wherein the distal clip (2) is fastened to the tie rod (4) and the proximal clip (3) slides with respect to the tie rod (4);
- an abutment element (5) for the clips (2, 3), slidably accommodated inside the main body (1), said abutment element (5) being provided with a longitudinal passageway for the tie rod (4);
- a handle (7), connected to the main body (1), to maneuver clips (2, 3), abutment element (5), and tie rod (4) ;
**characterized in that** the distal clip (2) is provided with at least one proximal housing (22), and the proximal clip (3) is provided with both a distal housing (32) and a proximal housing (33), and wherein the tie rod (4) is positioned inside said housings (22, 32, 33) when the clips (2, 3) are inserted into the main body (1).

2. A percutaneous device (100) according to claim 1, wherein the housing (22, 32, 33) is a longitudinal groove obtained on the outer surface of the clip (2, 3) having a rectangular or square or triangular or U-shaped or C-shaped or crescent-shaped section, or the housing (22, 32, 33) is a longitudinal flattening.

3. A percutaneous device (100) according to claim 1 or 2, wherein the distal clip (2) is provided with a distal seat (21) wherein the tie rod (4) is fastened, and said distal seat (21) transversally crosses the distal clip (2), perpendicularly.

4. A percutaneous device (100) according to claim 3, wherein the distal clip (2) comprises a path for the tie rod (4) which starts from the seat (21) and continues along the housing (22), and in wherein said path occupies half of the length of the distal clip (2).

5. A percutaneous device (100) according to any of the preceding claims, wherein the proximal clip (3) is provided with a proximal seat (31) in which the tie rod (4) is slidably inserted, and said proximal seat (31) is a through hole which transversely crosses the proximal clip (3), obliquely.

6. A percutaneous device (100) according to claim 5, wherein the proximal clip (3) comprises a path for the tie rod (4) which starts from the distal housing (32), continues into the seat (31) through the proximal clip (3) to exit from the opposite side and continue along the proximal housing (33), said path occupies the entire length of the proximal clip (3).

7. A percutaneous device (100) according to any of the preceding claims, wherein the clips (2, 3) and the tie rod (4) are made of resorbable material.

8. A percutaneous device (100) according to any of the preceding claims, wherein the tie rod (4) has a circular and/or flattened section.

9. A percutaneous device (100) according to any of the preceding claims, wherein the tie rod (4) is provided with a predetermined breaking point (44) and/or a radial space proximally with respect to the proximal clip (3).

10. A percutaneous device (100) according to any of the preceding claims, wherein the handle (7) comprises a handle body (71) provided with a track (73) in which an operating lever (72) is slidably accommodated, being integral with the main body (1) and sliding on the tie rod (4), and wherein said operating lever (72) comprises a lever stop (725) adapted to abut against a tie rod stop (45) so as to become integral with the tie rod (4), and wherein said handle (7) comprises a handle stop (75) adapted to abut against the operating lever (72) to stop the retraction thereof.

11. A percutaneous device (100) according to claim 10, wherein the tie rod (4) is provided with a traction element (45) or with a traction wheel (47).

12. A percutaneous device (100) according to claim 10 or 11, wherein the operating lever (72) comprises a cutting button (723) provided with a blade (724) adapted to proximally sever the tie rod (4).
